# EUROPEAN PATENT APPLICATION

(11) **EP 0 545 500 A1**
(43) Date of publication of application: **09.06.1993**
(21) Application number: 92203722.1
(22) Date of filing: 02.12.1992
(51) Int. Cl.: A61B 5/14, B01L 3/00

(54) **Capillary collection devices**

(30) Priority: 05.12.1991 US 803251
(71) Applicant: EASTMAN KODAK COMPANY, Rochester, New York 14650-2201 (US)
(72) Inventor: Columbus, Richard Lewis, Rochester, New York 14650-2201 (US); Palmer, Harvey John, c/o Eastman Kodak Company, Rochester, New York 14650-2201 (US)
(74) Representative: Phillips, Margaret Dawn

(57) **Abstract**

In known capillary collection devices, the collection rate tends to be relatively slow. Furthermore, a barrier is often need to separate two component phases of a liquid which has been collected and then centrifuged to separate it into its two component phases. Described herein is a device (10) and method for filling a capillary collection device with fill rates in excess of those obtained by a device having only a capillary spacing (d₁) between opposed walls (22, 24). Improved filling of the device (10) and maintenance of phase separation of the liquid is achieved by including a wettable foam material (40) positioned in the device between the walls (22, 24). The foam material (40) is chosen to have an open-cell structure and has extreme high porosity and filamentary connections between cells which are free of cell membranes between filaments.

## Description

The invention relates to a capillary devices, and is more particularly concerned with a device and method for collecting a liquid by capillary attraction, and preferably separating such liquid into its separate phases for removal of one or both phases.

In the field of capillary collection devices, attempts have been made with varying degrees of success to draw in body fluids by capillary attraction, from a source such as a finger prick, or an eyelid in the case of tear fluid. Generally, such devices rely on capillary transport created by a capillary spacing throughout the storage volume, as shown in, for example, US-A-4 136 036 and US-A-4 269 197. Once collected, the body fluid can be further processed. In the case of blood, phase separation is achieved by centrifugation and serum or plasma is collected from the device. In the case of tear fluid, the liquid is expelled under pressure.

Although such devices have functioned for their intended purpose, they have experienced some drawbacks. First of all, there is a limit to how much volume can be collected without the danger of gravity causing either a halt to the process, or spillage if the device is oriented vertically. Second, they have a rather slow rate of fill (typically, 10s are required for a volume of 60µl). Still further, in the case of blood collection and separation, it has been necessary to provide some way of physically blocking the remixing of the two phases during serum or plasma removal. The technique described in US-A-4 136 036 includes a gel of a specific gravity such that the gel locates itself between the two phases during centrifugation. Such a requirement for a gel adds to the expense and the complexity of the device.

Thus, it has been a problem prior to this invention to provide capillary collection vessels with a faster rate of collection, and in the case of blood collection and separation, one that does not need a gel-type physical barrier to maintain phase separation after it occurs.

It is therefore an object of the present invention to provide a capillary collection device which overcomes the above-noted problems through the addition of a particular kind of foam in at least a portion of the capillary transport zone.

More specifically, in accordance with one aspect of the invention, there is provided a capillary device comprising:-
an inlet aperture;
an outlet aperture; and
a chamber connected to the inlet aperture and the outlet aperture, the chamber being defined by spaced-apart walls at least two surfaces of which are spaced a distance no greater than that which will provide a capillary flow of liquid introduced in the chamber;
characterized in that the chamber includes in at least a portion adjacent to the inlet aperture, an open-cell foam of a compressible solid material wettable by the liquid, the porosity of the foam and the shape and size of interconnections between its cells being sufficient to provide a capillary influx of liquid placed at the inlet aperture which is faster than the capillary influx would be in the absence of the foam.

In accordance with a second aspect of the present invention, there is provided a liquid transport device for controlled fluid flow comprising:-
a liquid transport and storage chamber which is defined by spaced-apart walls at least two surfaces of which are spaced a distance no greater than that which will provide a capillary flow of liquid introduced in the storage chamber; and
means defining an inlet aperture and a separate vent aperture, the apertures being fluidly connected to the storage chamber;
characterized in that the storage chamber includes in at least a portion adjacent to the inlet aperture, an open-cell foam of a compressible solid material wettable by the liquid to be transported, the porosity of the foam and the shape and size of interconnections between its cells being sufficient to provide a capillary influx of liquid placed at the inlet aperture which is faster than the capillary influx would be in the absence of the foam.

In accordance with a third aspect of the present invention, there is provided a blood collecting and separating device having:-
a first aperture for collecting blood;
a second aperture through which a non-cellular phase can be removed; and
a chamber in which blood separates into a cellular phase and a non-cellular phase under the influence of centrifugal force, the chamber having a cellular phase collecting section and a non-cellular phase collecting section;
characterized in that an open-cell, wettable foam material is positioned within the chamber;
and in that the foam material comprises the sole means immobilizing an interface between the cellular phase collecting portion and the non-cellular phase collecting portion.

In accordance with a fourth aspect of the present invention, there is provided a blood collecting and separating device having:-
a first aperture for collecting blood;
a second aperture through which a non-cellular phase can be removed; and
a chamber extending from the first aperture and including a portion in which blood separates into a cellular phase and a non-cellular phase under the influence of centrifugal force, the chamber having a cellular-phase collecting section and a non-cellular phase collecting section, and is defined at least in a portion by two opposing generally solid surfaces of a plastic material spaced a capillary distance apart so that blood is attracted into the chamber by the capillary force alone of the capillary distance,
characterized in that the chamber further includes in at least a portion extending from the first aperture, an open-cell foam of a compressible, wettable solid material having a porosity, and shape and size of interconnections between its cells sufficient to enhance the capillary influx of blood placed at the first aperture compared to the influx of the extending portion lacking the foam, the foam being further effective to substantially totally release blood or blood components contained therein to a pipette subjected to a controlled aspiration by an aspirating device at a rate not exceeding 20µl/s.

In accordance with a fifth aspect of the present invention, there is provided a method of separating serum or plasma from whole blood comprising the steps of:-
a) collecting whole blood via capillary attraction into an open-cell foam of a compressible solid material wettable by the blood, the porosity of the foam and the shape and size of interconnection between the cells of the foam being sufficient to create an enhanced capillary draw on the blood compared to the draw rate in the absence of the foam,
b) separating the blood into its cellular phase and its non-cellular phase by spinning the foam about an axis while collecting the two separate phases in the foam, and
c) preferentially withdrawing one of the two phases from the foam.

Accordingly, it is an advantageous feature of the invention that the fill rate of a capillary collection device is enhanced over that heretofore available, while allowing collected liquid to be readily discharged.

It is another advantageous feature of the invention that such a device can be used to collect and separate the phases of whole blood without using a gel-type physical barrier to maintain the phases separate during removal of the non-cellular component.

A related advantageous feature is that much larger capillary volumes are collectable than has been feasible with conventional capillary devices.

For a better understanding of the present invention, reference will now be made, by way of example only, to the accompanying drawings in which:-
Figure 1 is a fragmentary sectional side elevational view of one embodiment of the device constructed in accordance with the present invention, showing its use to collect blood from a finger prick;
Figure 2 is a front elevational view of the device shown in Figure 1;
Figure 3 is a view similar to that shown in Figure 2, but illustrating a second embodiment of the present invention;
Figure 4 is a sectioned view taken generally along the line IV-IV of Figure 1, but only for the embodiment shown in Figure 3;
Figure 5 is a fragmentary sectioned side elevational view similar to that shown in Figure 1, but illustrating a third embodiment of the present invention;
Figure 6 is an enlarged microphotograph of the microstructure of a foam material which is useful with the invention taken at 150X magnification;
Figures 7A, 7B, 8A and 8B are graphs showing the improvement in fill rate achieved by the foam of the invention compared to the same device lacking the foam;
Figure 9 is a plot of fill rates as they vary with hematocrit;
Figures 10 and 11 are sectioned elevational views similar to that shown in Figure 1, but illustrating a fourth embodiment of the present invention particularly adapted to collection and separation of whole blood;
Figures 12 and 13 are plan views of a fifth embodiment of the present invention, the device being shown partially exploded in Figure 12;
Figure 14 is similar to Figure 13 but illustrates a sixth embodiment of the present invention;
Figure 15 is an isometric view of automated apparatus useful with the invention; and
Figure 16 is a fragmentary plan view taken horizontally through sensor 240 of Figure 15 to show its use with device 10E on the centrifuge rotor.

The invention is hereinafter described by reference to preferred embodiments wherein a particular foam material is described for collection of tears or whole blood, when the foam material is added to a capillary transport storage chamber of particular configurations. In addition, the invention is useful regardless of the particular composition of the foam material, of the type of liquid collected, and of the shape of the storage chamber, provided the foam material is wettable and has pores that are formed as defined here.

Orientation or directions such as "front", "back", "upper" and "lower" refer to relative orientations during the preferred usage of the device, particularly regarding usage during flow of liquid into the device.

As used herein, the "non-cellular phase" when applied to blood refers either to serum or plasma. It is serum if there is no anti-coagulant present, and otherwise it is plasma if an anti-coagulant is present (to keep the fibrinogen from being removed during phase separation). Anti-coagulants can be present, for example, by pre-coating them on the sidewalls of the container.

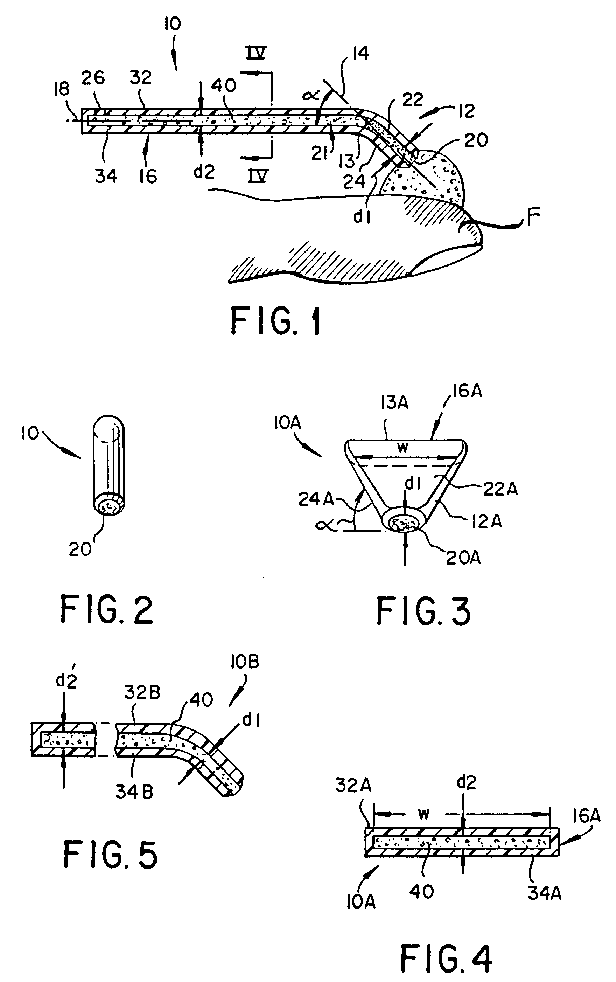

An overall configuration of a device 10 constructed in accordance with the present invention is shown in Figure 1. This configuration is preferably similar to that described in European application no. 92 200 940.2 filed on 2 April 1992, and published as EP-A-0 508 530, entitled "Gravity Assisted Collection Device." The device 10 comprises two portions 12 and 16, which join together at junction 13. Portion 12 is angled to portion 16 to form an angle α, which can be from 10 to 80° to allow portion 12 to be tipped out of the horizontal plane for blood collection from finger F. Axis 14 of portion 12 forms such angle α with the general axis 18 of portion 16, axis 14 being inclined out of the horizontal.

Portion 12 comprises an inlet aperture 20, and a chamber 21 defined in part by opposing walls 22 and 24 spaced-apart a distance d₁ no greater than that which will support capillary flow into the chamber from aperture 20. (Chamber 21 extends the full length of the device to aperture 26.) Most preferably, d₁ does not exceed 0.2cm for blood. (The value will vary, depending on the liquid collected, as is well-known). Preferably, d₁ is considerably less than this, for example, 0.1cm, for maximized flow control.

Portion 16 comprises a vent aperture 26 located preferably at the rear, in this embodiment, and wall portions 32 and 34 also preferably spaced apart a distance d₂ which is a capillary distance. Wall portions 32 and 34 thus define a chamber within portion 16 of device 10.

Together, the chambers of portions 12 and 16 comprise a collection and storage chamber for the liquid of choice. Because of the angle α between the two portions, portion 16 can be oriented horizontally, thus preventing gravity from defeating the capillary flow as it eventually will if angle α were zero and portion 16 continued with the same inclination to the horizontal as portion 12.

The collection and storage chamber formed by portions 12 and 16 in turn can form two sections, if the liquid collected is a two-phase liquid. For blood collection, the front section defined by portion 12 and the first part of portion 16 adjacent junction 13 becomes the non-cellular phase-collecting section, and the latter part of portion 16 where vent aperture 26 is located, becomes the cell-collecting section, at least in this embodiment. The exact demarcation between the two sections depends upon the hematocrit value, and the total volume collected.

As noted in EP-A-0 508 530, device 10 can have several alternative overall shapes. It can be a tube its entire length, as shown in Figure 2. In that case, dimension d₂ can equal or exceed d₁, and both are diameters.

Alternatively, as shown in Figure 3, it can have a flattened shape. Parts similar to those previously described bear the same reference numeral, to which the distinguishing suffix "A" is appended. Thus, devices 10A comprises a first portion 12A that has a generally circular aperture 20A and spaced-apart walls 22A and 24A which provide a capillary spacing d₁. Portion 16A joins with portion 12A at junction 13A, preferably again with a capillary spacing between its opposing wall portions 32A and 34A, as shown in Figure 4, as in the case of the previous embodiment. However, both portions 12A and 16A are flattened out to provide an internal spacing dimension W which is transverse to and exceeds the value of d₁ and d₂, to enhance the volume of the device. W can be and preferably is greater than a capillary distance. This however will not destroy the capillary flow into portion 16A if d₂ is still a capillary distance. The overall appearance then is one of a "wedge", as noted also in Figure 4.

Thus, for example, W can be 1.3cm so that the total volume of device 10A is 500µl.

For ease in shaping device 10 or 10A, the walls are preferably formed of a plastic material, for example, nylon, polyethylene, or even polypropylene. Any solid liquid impermeable plastic material will suffice, in this invention, even that which is not very wettable by the liquid of choice, because of the inclusion of the foam as described hereinafter.

Alternatively, as shown in Figure 5, the portion construed for horizontal disposition can have a spacing d₂' between the opposed walls that exceeds a capillary distance, again because of the foam added as described below. Parts similar to those previously described bear the same reference numeral to which the distinguishing suffix "B" is appended. Thus, device 10B is constructed exactly as shown in Figure 1, except that spacing d₂' of opposed walls 32B and 34B exceeds a capillary distance.

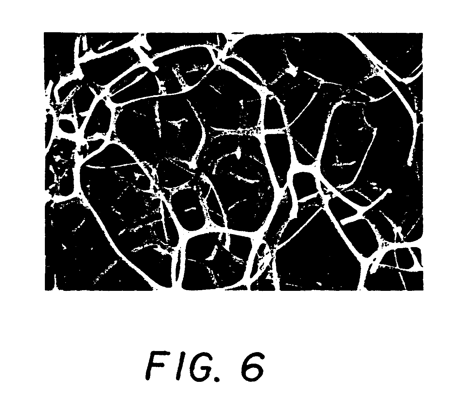

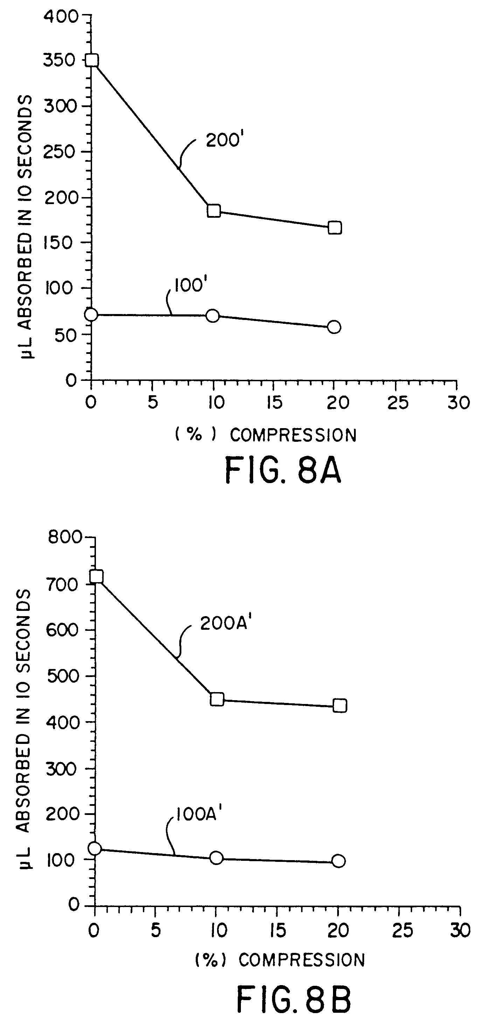

In accordance with the invention, at least a portion of the collection and storage chamber formed by portions 12 and 16, or 12A and 16A and preferably at least the front portion, is filled with an open-cell foam 40 of any compressible solid material which is a) wetted by the liquid to be collected and transported within the device, and b) has a porosity and interconnections between the cells of the foam which are sufficient to provide a capillary influx of liquid placed at the inlet aperture, which exceeds that which occurs in the absence of the foam. It has been found that a particular type of compressible open-cell foam is particularly able to provide these properties. It is any foam the cells of which form an isotropic network, when uncompressed, of very fine filaments of generally uniform thickness, as shown in Figure 6, and of dimensions and void volume described below. As described in US-A-4 929 969 entitled "Ink Supply Construction and Printing Method for Drop-On-Demand Ink Jet Printing", the preferred material, shown in Figure 6, is a form that is innately porous so as to have no cell "windows". The foam is a purely skeletal, three-dimensional, network formed without membranes in the network interstices, and hence with no "windows". The three-dimensionally interconnected skeletal filaments of material according to the present invention, provide and maintain uniform interstices to improve blood storage and delivery. In contrast to previous foams which are reticulated by burning techniques, the material of the present invention provide a significantly higher void volume, with no blocked, or partially blocked interstices and have no residual "burned-cell-wall" debris. This results in significant improvements as to the amount of blood which is storable in, and deliverable from given volumes of materials of the present invention. (A contrasting unacceptable material is reticulated polyurethane foam which has partially blocked interstices creating "windows", shown in Figure 8 of US-A-4 929 969.)

The interconnecting filaments have a relatively large length to width ratio, for example, in the order of 10 to 1 greater, and preferably are not thicker than 15µm. The innate skeletal nature of foam-type masses of such a foam material yields a low bulk density and large void volume because of the high percentage of their volume comprising interstitial voids. In these aspects, the materials can be characterized as having in their utilized condition within the device of the invention (that is uncompressed or compressed condition):
(i) a void volume greater than 90%, preferably greater than 95% and most preferably greater than 98%; or
(ii) a bulk density less than 24kg/m³ (1.5lbs./ft³), preferably less than 16kg/m³ (1.0lbs./ft³).

In regard to cell size, uncompressed materials according to the present invention can be characterized as having a relatively uniform cell size, with a relatively small percentage of voids significantly smaller than the average cell size. More particularly the materials can be characterized as having:
(i) an average cell size in the range of 25µm to 200µm, preferably in the range of 50µm to 175µm, most preferably having a majority of the cells in the size range of 140µm to 160µm; and
(ii) a cell size uniformity such that at least 95% of the cells have a size larger than 0.67 times the average cell size, preferably at least 97.5% or the cells have a size larger than 0.67 times the average cell size and most preferably at least 99.5% of the cells have a size large than 0.67 times the average cell size.

Other dimensional properties and variations can be found in US-A-4 929 969.

In regard to composition of foam materials, it is preferred that such materials be relatively inert vis-a-vis the body liquid stored, and not swell nor leach.

One highly preferred group of materials, with respect to their composition as well as other aspects described above, are the thermoset foam materials described in US-A-4 929 969 comprising preferably more than 80% melamine-formaldehyde condensate.

This particularly preferred group of melamine-formaldehyde condensate foams comprise a plurality of mutually connected, three-dimensionally-branched webs (or filaments). As described in US-A-4 929 969 the foam structures desirably have:
1. a mean length to width (diameter) ratio in order of 10:1 or greater; and
2. a web or filament density in the order of 1100kg/m³ (1.10g/ml) or greater.
Webs which are too short (that is in which the length to diameter ratio is too low) can decrease the large void volume characteristic preferred for maximizing ink storage.

As used herein, a foam material is "wettable" by the liquid being collected if that material (preferably when unfoamed) forms a contact angle with that liquid that is less than 90°.

Plastic foams which are "non-wettable" by the transported liquid as that term is defined herein, include polystyrene, polyethylene, polypropylene, some poly(vinyl chlorides), and thermoplastic polyester.

Also as used herein, the "substantial releasing" of the liquid from the material means, the release of at least 85% of the volume of the liquid contained in the foam material when the liquid is subjected to a controlled application of a partial vacuum or a partial pressure different from ambient conditions. A "controlled application" means, at a gradual rate, for example, between 1.0µl/s and 20µl/s. That is, the foam must readily release the liquid collected, for further use (for example, blood plasma released for diagnostic testing.)

Melamine foams of the kind described above are manufactured by BASF Aktiengesellschaft, Federal Republic of Germany (BASF Corp., Chemical Div., Parsippany, New Jersey), and sold under the tradename BASOTECT® by that company or under the tradename WILTEC® by Illbruck Schaumstofftechnik, Leverkusen, West Germany (Illbruck USA, Minneapolis, Minn.). These foams are commonly marketed for heat and sound insulation in buildings, vehicles and larger containers. Other known uses are as shock absorption packaging, bandages, cleaning materials and soil treatment, or as in US-A-4 929 969, storage of ink.

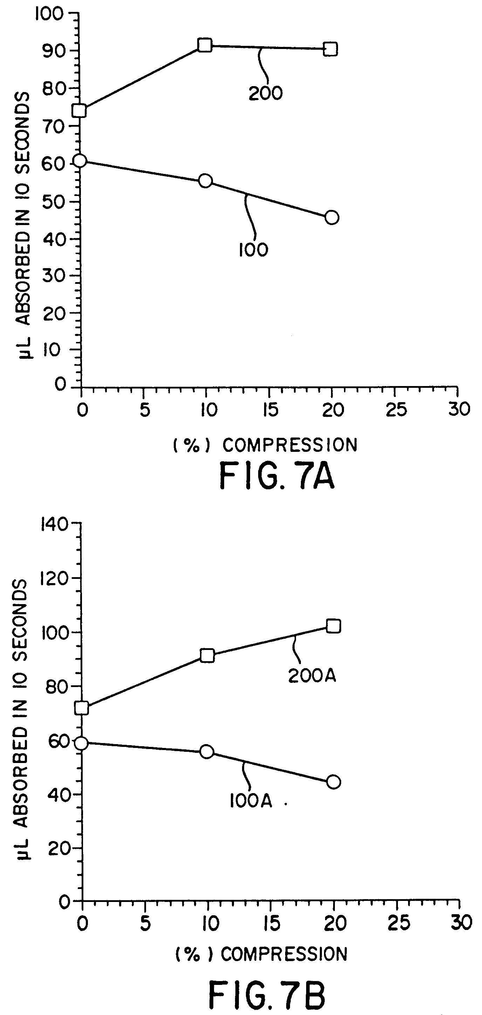

The foam can be confined within the storage volume of the device either substantially uncompressed in all three orthogonal directions, or compressed in any one or all three directions. For walls 22, 24 and 32, 34 (device 10) which are plastic as noted, the presence of compression does not alter the fact that the fill rate is enhanced. This is more readily apparent from Figures 7A and 7B, wherein whole blood and water, respectively, are tested.

Curves 100 and 100A, respectively show the fill rate for the device of the invention without any foam in the storage volume. Curves 200 and 200A, on the other hand, illustrate the same device but with the above-described foam at various % (by volume) compression, filling the storage volume. The compression occurred only in the d₁ and d₂ directions, that is, between the capillary-spaced walls. The inside surfaces of the walls were flat plates (simulating either section 12 or 16 of the device) comprising "Nylon 101"™ obtained from Zytel. The "% compression" achieved by curves 100 and 100A is not a "compression" but represents the change (decrease) in capillary spacing of d₁ or d₂ needed to achieve the noted compression when the foam (curves 200 and 200A) was present. The foam used was melamine foam obtained from Illbruck under the trademark "Wiltec" with 140µm overage cells and 95% void volume. The test protocol was in the case of Figures 7A and 7B to mount such plates in their spaced-apart configuration, with or without the foam, vertically on a mechanical arm set to move at a constant speed down until the sandwich just contacted a supply of test liquid. (The empty sandwich was previously weighed.) At this point, capillary attraction proceeded to draw in liquid and a stop-watch was started. After 10s, the sandwich was removed from the source of liquid and the amount of drawn-in liquid weighed. Volumes were calculated.

The results show the rate of fill (that is, the volume per 10s of fill time) for a capillary chamber oriented substantially vertically only - the orientation which inlet portion 12, Figure 1, approximates.

As noted in the Figures, it matters not whether the liquid drawn in is water (dyed for visibility) or whole blood, or what if any compression was present - the rate of fill was always faster when the foam was present. (Evidently there is a maximum compression % beyond which the fill rate will drop below the fill rate available without foam, when the cells become so closed off as to hinder flow. Although that maximum has not been determined, it is believe it is not reached until well beyond 60% compression, considering the 95% void volume provided by the preferred foam).

The behavior of curves 100 and 100A to produce a reduced fill rate as the gap is decreased, is well-known and is due to the fact that the resistance created by large surface opposed plates forming the capillary, overcomes the increased capillary attraction created by the reduced gap.

Surprisingly, however, when the foam is compressed by at least 2%, curves 200 and 200A show an increase in the fill rate over a zero % compression notwithstanding the decrease in fill rate which would be expected for a decrease in gap spacing from the results of curves 100 and 100A (no foam present). The exact mechanism for this increased fill rate due to the foam compression is not understood. It may arise from the fact that the cell compression occurs more at the solid surface of the walls, and that the compression draws the liquid into better contact with the wall surface than would be the case in the absence of any foam whatsoever.

Also for reasons which are not understood, this enhanced fill rate, achieved by some compression, does not occur if the orientation of the chamber and the foam within it, is horizontal (as in the portion 16 of device 10, Figure 1). This is more readily apparent from the curves of Figures 8A and 8B.

The experiment of Figures 7A and 7B was repeated, except the assembled sandwich was maintained in a horizontal orientation, with or without foam. This sandwich was moved horizontally along the top surface of a smooth block of "Teflon"™ plastic, and brought into timed contact with a large (1ml) pool of liquid which had been placed on the "Teflon" plastic.

Volumes were determined by weighing and calculating as noted above. In the horizontal orientation, compression of the foam only served to reduce the fill rate that is otherwise available at zero % compression. However, even that reduced rate is well above what is achieved for the same gapping between plates without the foam, due to the marked improvement at zero % compression (700%) for both curves 200' and 200A', compared to the control curves 100' and 100A' (without foam).

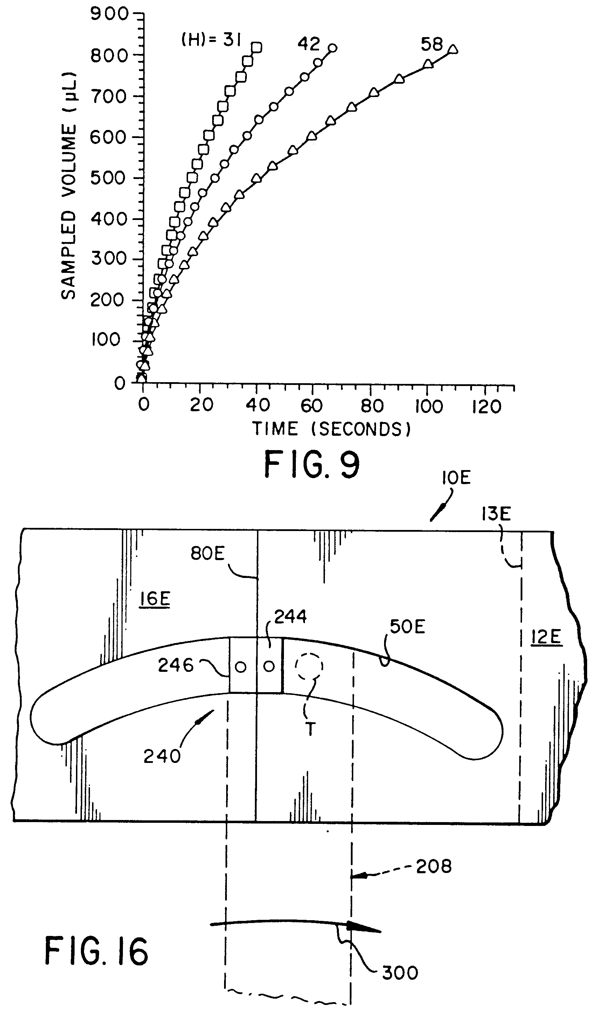

The practical use of the invention is well illustrated in Figure 9, wherein a horizontally disposed reservoir was used, as for Figures 8A and 8B. The experiment was the same as for Figures 8A and 8B, except that the foam was 1.3mm thick in its uncompressed state, and was then compressed 10% in all three cases. Three such horizontal sandwiches were formed, and used to absorb whole blood having respectively, a hematocrit (H) of either 31, 42, or 58. The rate of fill is shown in Figure 9, where the total volume between the solid surfaces (in the absence of foam) was larger than 900µl. In these tests, volumes were determined by video-recording the advancing wavefront of the blood, as a length of the volume being filled, knowing the correction factor needed (0.96) for the space occupied by the foam filaments.

The collection of more than 800µl in only 40s, for H = 31, is very surprising, compared to the fill rates, and volumes, that are available in conventional capillary devices. In fact, that much volume cannot be achieved by any conventional capillary device for any length of time.

Additional tests have shown that a similar enhancement in fill rate occurs when using for the opposed plates a plastic besides "Nylon". That is, the addition of the foam of the invention gives an enhanced fill rate in such a case.

The test noted above on dyed water indicate that the invention is equally useful on liquids having a higher water content than whole blood, for example, tear liquid from the eye.

In the embodiments described so far, the collected liquid can be removed from the device by applying either positive pressure to the air vent 26 or suction to the collection orifice 20.

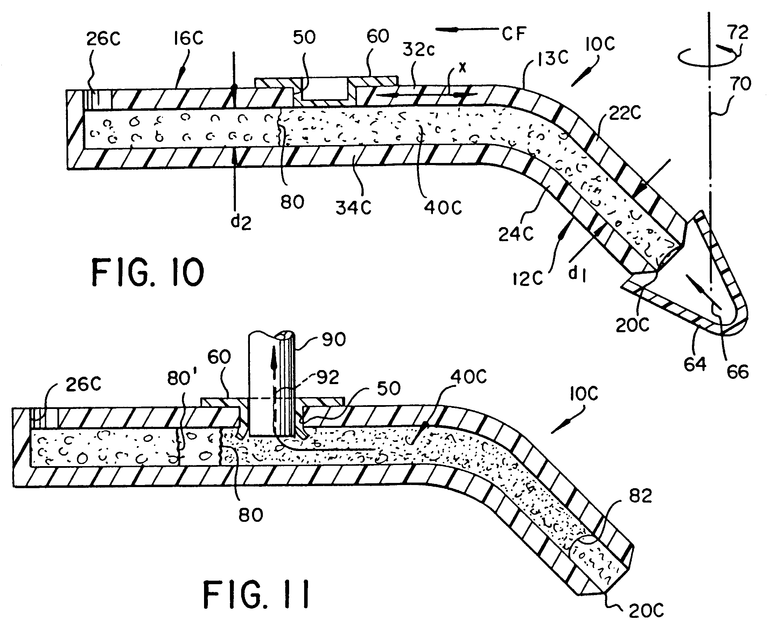

The liquid removal need not be done in this fashion. Instead, as shown in Figures 10 and 11, an additional pipetting aperture can be supplied in the rear portion of the device. Parts similar to those previously described bear the same reference numeral, to which the distinguishing suffix "C" is applied.

Thus, device 10C comprises a first front portion 12C, and a second rear portion 16C joining it at juncture 13C. As in the previous embodiments, inlet aperture 20C is provided in portion 12C, vent aperture 26C is provided in portion 16C, and the foam material 40C of the invention is provided within the chamber defined by the opposing walls of portions 12C and 16C, for example walls 22C and 24C, either compressed or uncompressed. Most preferably, the foam extends the full distance from aperture 20C to the vent aperture 26C, and is compressed from 5 to 10% between walls 22C and 24C only, portion 12C only. (This can be readily achieved by increasing d₂ of portion 16C over d₁ of portion 12C by from 5 to 10%. Still further, most preferably device 10C is wedge-shaped as in the embodiment of Figures 3 and 4.

Unlike the previous embodiments, an additional aperture 50 is provided anywhere in portion 16C, but most preferably in upper wall portion 32C rather than lower wall portion 34C. This aperture is sized to receive an operating pipette, as will be clearer hereinafter. Its relative position x, Figure 10, between junction 13C and vent aperture 26C depends upon the hematocrit of the whole blood which is to be collected, which can vary. Thus, it needs to be located forward of the expected phase division of cells and serum or plasma after phase separation occurs as described below. For a total volume of 500µl where angle α, Figure 3, is 45°, distance x is preferably 4.3cm.

A self-adhering seal 60 is applied to aperture 50 of a material capable of being punctured by a pipette.

In use, aperture 20C is inserted into a finger prick in the manner shown in Figure 1, and whole blood is rapidly collected until the advancing meniscus fills the storage chambers including the region under or adjacent aperture 26C. At this point, a seal of a material similar to seal 60 (not shown) is placed over vent aperture 26C, and preferably a seal cap 64 is placed, in the direction indicated by arrow 66, over aperture 20C.

Thereafter, device 10C is positioned in any suitable centrifuge so as to spin it about a spin axis 70, in the direction shown by arrow 72, creating a centrifugal force shown by the arrow CF. The force CF is thus effective to cause phase separation within foam 40C, forcing the blood cells to migrate to the back end of portion 16C, as shown by the "circles". The cell sizes within the foam described above are adequate for this purpose. The phase division 80 between the cells and serum or plasma occurs in back of aperture 50, when aperture 50 is properly located for the maximum hematocrit expected to be encountered. For example, phase separation on a volume of 500µl is achieved while spinning at 400g, in 3min, and the serum or plasma meniscus 82 is now located removed from inlet 20C, as shown in Figure 11.

At this point, an aspirating pipette 90 is inserted through seal 60 and into aperture 50, and a vacuum is drawn, as indicated by arrow 92. By using a vented cap 64, air can come into the orifice 20C to replace the serum or plasma which is withdrawn. Because of the ready liquid releasibility of foam 40 as described above, at least 85% by volume, and preferably substantially all the separated serum or plasma can be withdrawn in this fashion, by any aspirating device.

In contrast, the cells collected between boundary 80 and vent aperture 26C do not move forward into pipette 90 because air vent 26C is sealed, so that air cannot intrude into the foam volume occupied by cells.

Optionally, water-soluble reagents can be present in the foam, preferably dispersed throughout the portion 12C of the device, which interact with the liquid which is collected in the foam. For the preferred use with blood, the reagent preferably includes either a coagulant or an anti-coagulant, depending on whether serum or plasma is desired after centrifugation. Useful coagulants include all those listed in US-A-4 994 192, and particularly polylysine or poly(2-aminoethyl methacrylate hydrochloride). Of the anticoagulants, particularly preferred are one or more selected from the group consisting of ethylenediaminetetraacetic acid disodium and tripotassium salts, sodium oxalate, sodium citrate, buffered sodium citrate, sodium heparin, lithium heparin, ammonium heparin and lithium iodoacetate. Such reagents preferably are present from 0.1mg/ml of foam volume up to the amount needed to saturate the liquid in the foam. However, the amount used should be less than that which, when dry, blocks off liquid access to the void volume.

Other reagents that can be dispersed on or in the foam include microbial inhibitors such as sodium fluoride and/or mixtures of that and potassium oxalate or rodium fluoride and thymol.

The advantage of dispersion of the reagents within the foam is that it provides a much more rapid dissolution of the reagents than if they are on the sidewalls 22C or 24C only.

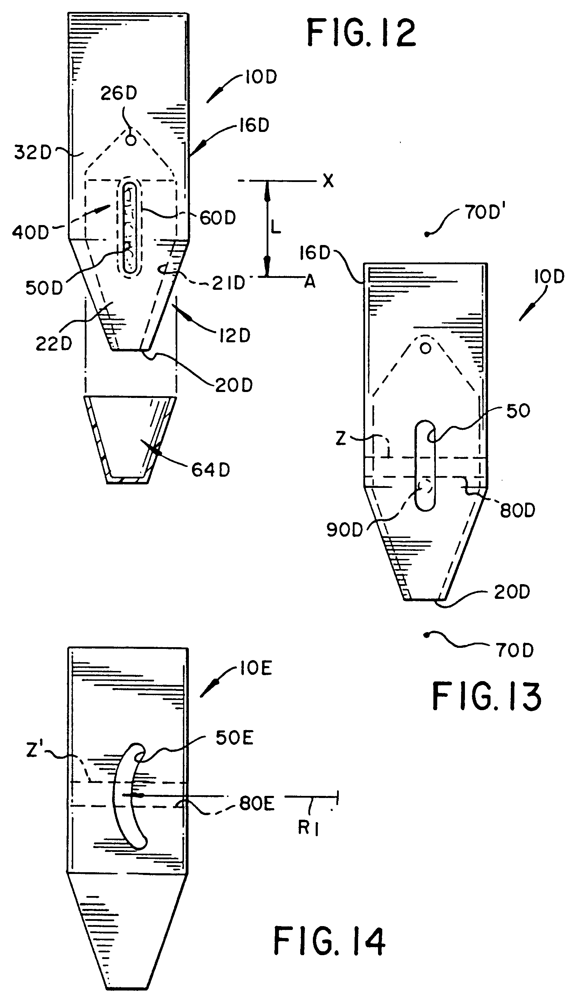

It is not necessary that the removal aperture be fixed at the "worst" case hematocrit location, for the invention. In fact, this fixed location is less desirable since it makes it likely that in many cases boundary 80 is in fact located at 80', Figure 11, leaving some serum or plasma uncollected after aspiration. Instead, Figures 12 to 15, a variable location can be provided. Parts similar to those previously described bear the same reference numeral, to which the distinguishing suffix "D" is applied.

Thus, Figure 12, device 10D is constructed as before, with an internal collection and separation chamber 21D extending from an inlet aperture 20D to vent aperture 26D, chamber 21D featuring opposed walls (of which only 22D and 32D are shown) creating a capillary spacing within chamber 21D, at least at front portion 12D, all as described heretofore. Additionally, foam 40D is included in part or all of chamber 21D, and if only part, in the front portion 12D and the front part of rear portion 16D, and a liquid removal aperture 50D provides air access to chamber 21D (when the seal 60D, shown in phantom, is perforated). Cap 64D is provided as before.

Unlike the previously-described embodiment, aperture 50D is not uniquely located to allow pipette access at only one location between, for example, position A and position X, so that only the most exactly selected hematocrit value will cause the phase boundary to closely align with the pipette. Instead, aperture 50D is provided with a length L which is greater than the outside diameter of the pipette tip insertable therein, by multiple units of that outside diameter. As a result, the pipette tip can be inserted at variable locations along the length L, depending on where the phase boundary ends up being. Positioning of the tip within the serum or plasma side of the phase boundary is then done either by visual observation, or automatically as described in the operation below.

Alternatively, elongated aperture 50D can be replaced with a series of circular apertures (not shown), and positioned along length L to approximate the continuum of locations better provided by aperture 50D. The total access provided by such a series of apertures provides an effective aperture length of variable access.

In operation, whole blood is drawn in at aperture 20D as for previous embodiments, until it reaches a location Z, Figure 13, preferably somewhere between positions A and X. Cap 64D is placed on aperture 20D, and then device 10D can be spun about axis 70D as described above. If the phase boundary 80D forms as shown, then the pipette which is brought in for aspiration simply occupies the dotted position 90D to remove the serum or plasma.

Optionally, the foam 40 in all the embodiments need not fill the back part of the compartment (not shown) since its function serves primarily for drawing in the liquid. The phase separation still occurs as described, whether or not foam is at a particular location.

Alternatively, spin axis can be disposed adjacent the end of portion 16D, shown as 70D'. In that case, the pipette position 90D must be on the opposite side of the formed boundary line 80D than is shown in Figure 13.

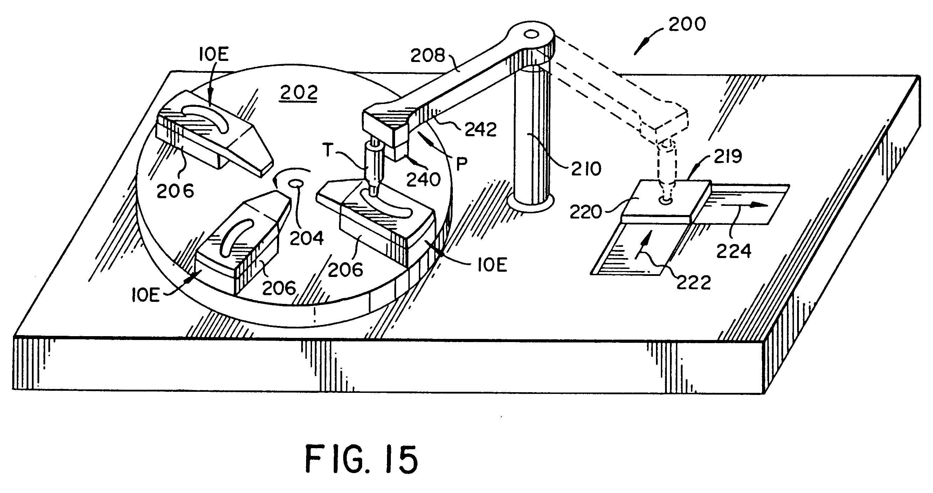

To detect the location of boundary 80D automatically, it is preferred, as will become apparent, that slot 50D be curvilinear rather than straight, Figure 14. Parts similar to those previously described bear the same reference numeral, to which the distinguishing suffix "E" is applied. Thus, device 10E is exactly the same as described for the embodiment of Figures 12 and 13, except that the liquid removal slot 50E is curvilinear, with a radius of curvature R₁. R₁ has a value which is substantially equal to the length of rotational arm 208 (Figure 15) by which a pipette P can be swung out over device 10E to be closer to, or farther away from, location Z', depending on the exact location of the phase boundary 80E.

Turning more specifically to the automated apparatus 200 of Figure 15, this preferably comprises a centrifuge rotor 202 mounted to rotate about axis 204 and driven by conventional motor means, not shown. Suitable clamps, not shown, hold a plurality, for example, four, collection devices 10E in place, of which only three are shown, on raised block 206. Pipette tips T are mounted on to aspirator arm 208, that is pivotally mounted on rod 210 constructed to move up and down also, on command, by a drive mechanism (not shown). Within rod 210, not shown, is a passageway that connects with the interior of tip T and with a conventional metering pump for pipette P. Such pump is effective to first generate a partial vacuum within tip T, and then a partial pressure when the tip is rotated (shown in phantom) to station 212 for dispensing the liquid onto or into any suitable container or test element 220. Container 220 is automatically advanced in the direction shown by arrow 222 to station 219, and removed, in the direction shown by arrow 224, by conventional pusher blades (not shown) to further processing stations.

The arc traversed by tip T as arm 208 is rotated is the same arc as is provided in the curvature of slot 50E. To sense exactly where along that arc tip T should stop and descend, an infrared sensor 240 is provided, as is better shown in Figure 16. Sensor 240, mounted on the underside 242 of arm 208 adjacent tip T, Figure 15, comprises, as is conventional, an infrared-emitting diode 244 and a sensor 246, Figure 16. Such a device easily detects the greater infrared absorption occurring in the cellular components which are to the rear of boundary 80E, here shown as a solid line, due to the heme content of that phase (in portion 16E of device 10E). As the sensor swings (to the right as shown) along arc 300 towards junction 13E, and portion 12E, the abrupt increase in reflection which occurs in the non-cellular phase (in front of boundary 80E) is detected and the arm 208 (shown in phantom) ceases its rotation. Tip T is then automatically depressed through any seal and into slot 50E, to remove the non-cellular phase by aspiration.

Following the removal by aspiration of the non-cellular components, the cellular components are contained for controlled disposal with the collection device.

The invention disclosed herein may be practiced in the absence of any element which is not specifically disclosed herein.

## Claims

1. A capillary device (10; 10A; 10B; 10C; 10D; 10E) comprising:-
an inlet aperture (20; 20A; 20C; 20D);
an outlet aperture (26; 26C; 26D); and
a chamber (21; 21D) connected to the inlet aperture (20; 20A; 20C; 20D) and the outlet aperture (26; 26C; 26D), the chamber (21; 21D) being defined by spaced-apart walls (22, 24; 22A, 24A; 22C, 24C; 22D) at least two surfaces of which are spaced a distance (d₁) no greater than that which will provide a capillary flow of liquid introduced in the chamber (21; 21D);
characterized in that the chamber (21; 21D) includes in at least a portion adjacent to the inlet aperture (20; 20A; 20C; 20D), an open-cell foam (40; 40C) of a compressible solid material wettable by the liquid, the porosity of the foam (40; 40C) and the shape and size of interconnections between its cells being sufficient to provide a capillary influx of liquid placed at the inlet aperture (20; 20A; 20C; 20D) which is faster than the capillary influx would be in the absence of the foam (40; 40C).

2. A liquid transport device (10; 10A; 10B; 10C; 10D; 10E) for controlled fluid flow comprising:-
a liquid transport and storage chamber (21; 21D) which is defined by spaced-apart walls (22, 24; 22A, 24A; 22C, 24C; 22D) at least two surfaces of which are spaced a distance (d₁) no greater than that which will provide a capillary flow of liquid introduced in the storage chamber (21; 21D); and
means defining an inlet aperture (20; 20A; 20C; 20D) and a separate vent aperture (26; 26C; 26D), the apertures (20, 26; 20A; 20C, 26C; 20D, 26D) being fluidly connected to the storage chamber (21; 21D);
characterized in that the storage chamber (21; 21D) includes in at least a portion adjacent to the inlet aperture (20; 20A; 20C; 20D), an open-cell foam (40; 40C) of a compressible solid material wettable by the liquid to be transported, the porosity of the foam (40; 40C) and the shape and size of interconnections between its cells being sufficient to provide a capillary influx of liquid placed at the inlet aperture (20; 20A; 20C; 20D) which is faster than the capillary influx would be in the absence of the foam (40; 40C).

3. A blood collecting and separating device (10; 10A; 10B; 10C; 10D; 10E) having:-
a first aperture (20; 20A; 20C; 20D) for collecting blood;
a second aperture (26; 26C; 26D) through which a non-cellular phase can be removed; and
a chamber (21; 21D) in which blood separates into a cellular phase and a non-cellular phase under the influence of centrifugal force (CF), the chamber (21; 21D) having a cellular phase collecting section and a non-cellular phase collecting section;
characterized in that an open-cell, wettable foam material (40; 40C) is positioned within the chamber (21; 21D);
and in that the foam material (40; 40C) comprises the sole means immobilizing an interface (80, 80'; 80D) between the cellular phase collecting portion and the non-cellular phase collecting portion.

4. A blood collecting and separating device (10; 10A; 10B; 10C; 10D; 10E) having:-
a first aperture (20; 20A; 20C; 20D) for collecting blood;
a second aperture (26; 26C; 26D) through which a non-cellular phase can be removed; and
a chamber (21; 21D) extending from the first aperture (20; 20A; 20C; 20D) and including a portion in which blood separates into a cellular phase and a non-cellular phase under the influence of centrifugal force (CF), the chamber (21; 21D) having a cellular-phase collecting section and a non-cellular phase collecting section, and is defined at least in a portion by two opposing generally solid surfaces (22, 24; 22A, 24A; 22C, 24C; 22D) of a plastic material spaced a capillary distance (d₁) apart so that blood is attracted into the chamber (21; 21D) by the capillary force alone of the capillary distance (d₁),
characterized in that the chamber (21; 21D) further includes in at least a portion extending from the first aperture (20; 20A; 20C; 20D), an open-cell foam (40; 40C) of a compressible, wettable solid material having a porosity, and shape and size of interconnections between its cells sufficient to enhance the capillary influx of blood placed at the first aperture (20; 20A; 20C; 20D) compared to the influx of the extending portion lacking the foam (40; 40C), the foam (40; 40C) being further effective to substantially totally release blood or blood components contained therein to a pipette (90; 90D) subjected to a controlled aspiration by an aspirating device at a rate not exceeding 20µl/s.

5. A device according to any one of the preceding claims, wherein liquid is readily released from the foam (40; 40C) under the controlled application of a differential pressure or vacuum.

6. A device according to any one of the preceding claims, wherein the foam (40; 40C) comprises a three-dimensionally branched network of fine filaments interconnected to form interstitial cells of a void volume of at least 90% of the volume of the foam when uncompressed.

7. A device according to any one of the preceding claims, wherein the foam (40; 40C), in its uncompressed state, has a bulk density of less than 24kg/m³ (1.5lbs/ft³).

8. A device according to any one of the preceding claims, wherein the foam (40; 40C) is substantially isotropic in its uncompressed state.

9. A device according to any one of the preceding claims, wherein the foam (40; 40C), in its uncompressed state, has an average cell size in the range of 25µm to 200µm.

10. A device according to claim 9, wherein the majority of the foam material cells have a size in the range of 50µm to 175µm when uncompressed.

11. A device according to claim 6, wherein the foam filaments have a length to width ratio in the order of 10 to 1 or greater, and a thickness no greater than 15µm.

12. A device according to any one of the preceding claims, wherein the foam (40; 40C) contains substantially no cell membranes between filaments.

13. A device according to any one of the preceding claims, wherein the foam (40; 40C) is substantially chemically inert with respect to blood.

14. A device according to claim 13, wherein the foam (40; 40C) contains substantially unmodified melamine-formaldehyde condensate which has been thermoset.

15. A device according to claim 4, wherein at least a portion of the non-cellular phase collecting section extends from the first aperture (20; 20A; 20C; 20D) and is inclined at an angle (α) to a portion of the cellular phase collecting portion so that the inclined portion can be disposed at an angle (α) raised from the horizontal to draw blood in,
and wherein the foam (40; 40C) is compressed in the inclined portion between the surfaces (22, 24; 22A, 24A; 22C, 24C; 22D) by at least 2% of its uncompressed volume so that the fill rate of the inclined portion is enhanced over the fill rate of the inclined portion using the foam (40; 40C) in an uncompressed state.

16. A device according to any one of the preceding claims, wherein the entire chamber (21; 21D) is substantially filled with the foam (40; 40C).

17. A device according to any one of the preceding claims, further including a liquid-removal aperture (50; 50D; 50E) in a portion of the chamber (21D) containing the aperture (26C; 26D) which is spaced away from the aperture (26C; 26D) a distance sufficient to confine the cellular phase collected from a whole blood sample collected in the chamber (21D) between the removal aperture (50; 50D; 50E) and the aperture (26C; 26D), when the whole blood sample is phase-separated by centrifuging.

18. A device according to claim 17, further including a puncturable seal (60; 60D) over at least the removal aperture (50; 50D; 50E).

19. A device according to any one of claims 1 to 16, further including one or more liquid-removal apertures (50D; 50E) providing access to the chamber (21D) at variable locations between the two apertures (20C, 26C; 20D, 26D), the operative length of the one or more removal aperture (50D; 50E) measured in a direction between the two apertures (20C, 26C; 20D, 26D) being greater than the outside diameter of a pipette tip (90; 90D) insertable into the removal aperture (50D; 50E) so that a pipette (90; 90D) can be inserted into the one or more removal apertures (50D; 50E) wherever the hematocrit value causes a phase boundary (82) to locate.

20. A device according to claim 19, wherein the one or more liquid-removal aperture (50E) is curvilinear.

21. A device according to any one of the preceding claims, further including a water-soluble reagent in the foam material capable of interacting with any aqueous liquid within the chamber (21; 21D).

22. A device according to claim 21, wherein the reagent is an anti-coagulant or a coagulant.

23. A device according to claim 22, wherein the reagent is an anticoagulant selected from the group consisting of ethylenediaminetetraacetic acid disodium and tripotassium salts, sodium oxalate, sodium citrate, buffered sodium citrate, sodium heparin, lithium heparin, ammonium heparin and lithium iodoacetate.

24. A device according to claim 22, wherein the reagent is a coagulant selected from polylysine and poly(2-aminoethyl methacrylate hydrochloride).

25. A device according to Claim 21, wherein the reagent reacts with blood and is dispersed throughout the part of the foam material which is first contacted by incoming liquid.

26. A method of separating serum or plasma from whole blood comprising the steps of:-
a) collecting whole blood via capillary attraction into an open-cell foam (40; 40C) of a compressible solid material wettable by the blood, the porosity of the foam and the shape and size of interconnection between the cells of the foam (40; 40C) being sufficient to create an enhanced capillary draw on the blood compared to the draw rate in the absence of the foam (40; 40C),
b) separating the blood into its cellular phase and its non-cellular phase by spinning the foam (40; 40C) about an axis (70; 70D, 70D') while collecting the two separate phases in the foam (40; 40C), and
c) preferentially withdrawing one of the two phases from the foam (40; 40C).

27. A method according to claim 26, wherein the step of withdrawing comprises the step of removing at least 85% of the non-cellular phase from the foam (40; 40C).

28. A method according to claim 26 or 27, wherein the step c) comprises inserting a pipette (90; 90D) into the foam (40C) at a variable location determined by the location of the phase boundary (82) between the cellular phase and the non-cellular phase and applying a partial vacuum while venting the portion of the foam (40C) holding the non-cellular phase at a location opposite to the phase boundary (82).

29. A method according to claim 28, wherein the inserting step comprises sensing the boundary's location with an infrared beam and automatically moving a pipette (90D) in response to the sensing step to position it in the non-cellular phase adjacent to the boundary (82).
